# EUROPEAN PATENT SPECIFICATION

(11) **EP 4 064 953 B1**
(45) Date of publication and mention of the grant of the patent: **25.10.2023**
(21) Application number: 19872271.2
(22) Date of filing: 25.11.2019
(51) Int. Cl.: A61B 5/16, A61B 5/395

(54) **A SYSTEM FOR MONITORING AUDITORY STARTLE RESPONSE**
SYSTEM ZUR ÜBERWACHUNG VON AUDITIVER SCHRECKREAKTION
SYSTÈME PERMETTANT DE SURVEILLER UNE RÉPONSE À UNE SURPRISE AUDITIVE

(43) Date of publication of application: 05.10.2022
(73) Proprietor: Cakmak, Yusuf Ozgur, 34700 Uskudar/Istanbul (TR)
(72) Inventor: Cakmak, Yusuf Ozgur, 34700 Uskudar/Istanbul (TR)
(74) Representative: Atalay, Baris
(86) International application number: PCT/TR2019/050992
(87) International publication number: WO 2021/107886

(56) References cited:
- CN-A- 109 223 006
- US-A1- 2015 289 813
- US-A1- 2019 008 435
- CHRISTIAN GRILLON ET AL: "Baseline startle amplitude and prepulse inhibition in Vietnam veterans with posttraumatic stress disorder", PSYCHIATRY RESEARCH., vol. 64, no. 3, 1 October 1996 (1996-10-01), pages 169-178, XP055721507, IE ISSN: 0165-1781, DOI: 10.1016/S0165-1781(96)02942-3 cited in the application

## Description

### Technical Field of the Present Invention

The present invention relates to a system whereby the auditory startle response of a subject is determined for diagnosis of PTSD.

### Background of the Present Invention

Post-traumatic stress disorder (PTSD) can develop in individuals exposed to intense trauma that threatens physical injury or death. People who develop PTSD endure chronic psychological distress by repeatedly reliving their trauma through intrusive, flashback memories which are frequently precipitated by the presence of cues associated with the traumatic event. Additional debilitating symptoms, such as depression and substance abuse usually occur alongside the re-experiencing and avoidance symptoms of the disorder. Over the decades several physiological and behavioral abnormalities associated with PTSD have been documented, including heightened autonomic arousal, exaggerated startle, abnormally low baseline levels of cortisol, smaller hippocampal volume and cognitive impairments based on impaired hippocampal and prefrontal cortical functioning (Zoladz and Diamond. Neurosci Biobehav Rev. 2013. 37:860-895).

Currently PTSD is diagnosed by heart rate variability, questionnaires such as CAPS-5 and the PTSD checklist and neuroimaging. However, heart rate variability has limited effectiveness and is still controversial while questionnaires are highly subjective and therefore can be unreliable.

Neuroimaging methods most widely used to diagnose PTSD are functional magnetic resonance imaging (FMRI) and magnetoencephalography (MEG). Neuroimaging has high accuracy but is very expensive to operate and has limited availability. Therefore, there exists a need for alternative biological diagnostic methods that are practical, accurate and inexpensive.

The presence of a physiological alteration accompanying a mental disorder, such as PTSD, can produce data that are more objective and more readily quantifiable than self-report data. For this reason, other biological markers, such as exaggerated startle response are being investigated as potential tools to diagnose PTSD. The startle response is a response to abrupt and intense stimulation, consisting of a rapid sequential muscle contraction with the likely purpose of facilitating the flight reaction and/or to protect the body from sudden attack. The amplitude of startle response is variable in a way that reflects variation in the internal state of the person, such as fear and anxiety, and so the startle reflex can be used as a probe of this internal state (Grillon and Baas. Clin Neurophysiol. 2003. 114:1557-1579).

As mentioned above, exaggerated startle is linked to PTSD and is also a DSM-5 diagnostic criterion for the disorder. The abnormal physiological startle response seen in trauma survivors may develop, along with PTSD, after traumatic exposure as a consequence of a progressive sensitization of the central nervous system (CNS). The exaggerated startle response is generally measured by recording the eyeblink reflex, cardiac acceleration and increased electrodermal conductivity. However, it can also be measured by pupillometry, which is the measurement of pupil size and reactivity. Pupil constriction is controlled by the iris sphincter muscle and pupil dilation is controlled by the iris dilator muscle. The iris sphincter is innervated by the parasympathetic nervous system, the part of the autonomic nervous system that is involved in homeostasis. The iris dilator muscle is controlled by the sympathetic nervous system, the part of the autonomic nervous system that is involved in arousal, wakefulness, and the fight-or-flight response. The dilation pathway is a subcortical pathway that starts at the hypothalamus and the locus coeruleus (LC) and connects to the iris dilator muscle. The pupil dilates after an arousing stimulus, thought, or emotion, for example a visual or acoustic stimulus (Mathôt. Journal of Cognition. 2018. 1:1-23).

Acoustic startle is evoked by short (50 ms) noises, usually broadband or white noise with a high intensity (90-110 dB). Startle stimuli can be delivered at any time to probe ongoing affective and mental processes. Electrical activity associated with contraction of the orbicularis oculi muscle can be detected with an EMG using two electrodes below one eye (Grillon and Baas. Clin Neurophysiol. 2003. 114:1557-1579; Shalev et al. Am J Psychiatry. 2000. 157:255-261).

Methods and devices utilizing exaggerated startle response as a tool to assist in diagnosing PTSD are present in the art. An example of such a method may be referred to as WO 2014/179671 (A1), which discloses a system and method for classification and diagnosis of patients suffering from anxiety disorders, such as PTSD, by measuring objective physiological measures, such as inter-heartbeat interval and skin conductance. The system comprises a stimulus delivery module that provides audio and visual, traumatic or non-traumatic stimulus to the subject. The system then measures various resulting physiological signals and extracts features from the physiological measures and a diagnosis is made by classifying the extracted features.

Another example may be referred to as US 2015/289813 (A1), which discloses a system and method for the biological diagnosis of PTSD comprising an electronic device equipped with a built-in or attachable camera, built-in or attachable flash or controllable light source, and a software application. The software application includes a method that records and monitors the diameter of an individual's pupil prior to and after the application of light, using the camera and flash in communication with the electronic device. In another embodiment, the method includes the use of emotionally-charged visual stimuli to increase the accuracy of the diagnosis. In still another embodiment, a heart rate monitor is used to monitor the individual's heart rate variability to further increase the accuracy of the diagnosis. In still another embodiment, the method also measures the auditory startle response by measuring the orbicularis oculi response of the individual by electromyography. The method analyzes the data collected and determines the likelihood of individual suffering from PTSD.

An alternative measure of exaggerated startle response is the post-auricular muscle response (PAMR). PAMR is a large sound-evoked vestigial muscle response that acts to pull the ear backward, which can be evoked by clicks or tone-bursts and can be measured on the skin surface over the muscle behind the ear by electrodes. Currently, PAMR is mostly used as a clinical tool to test hearing due to the speed and ease with which the response can be obtained (O'Beirne and Patuzzi. Hearing Res. 1999. 138:115-132).

An example of hearing-related use of PAMR may be referred to as WO 2012/129465 (A1) which discloses an arrangement for custom fitting a hearing prosthesis system, such as cochlear implant systems, to a patient to optimize its operation. The PAMR measurement determines a PAMR of the patient to an auditory stimulus signal. For example, the PAMR may include a PAMR amplitude growth function or a PAMR threshold stimulus level at which a PAMR is measured in the patient. Then a patient fitting module sets an operating characteristic of the hearing prosthesis system based on the PAMR response.

Another example may be referred to as WO 2007/136901 (A2), which discloses a device and method for objectively measuring tinnitus in human and animal subjects. The startle reflex is induced by exposing a subject to an alteration in a sound pattern otherwise qualitatively similar to the subject's tinnitus. The subject's acoustic startle response is obtained and used to determine whether the subject detected the alteration of the sound pattern. The device comprises a controller for selecting a primary sound pattern and selecting a reflex stimulus sound pattern, a generator for generating signals associated with the sound patterns selected by the controller, a transducer for converting the generated signals to the selected sound patterns and exposing the selected sound patterns to the subject and a response sensor for detecting a response by the subject to the selected reflex stimulus sound pattern.

However, recent studies have also shown that patients with PTSD exhibited normal acoustic startle amplitude, but showed a significant reduction in prepulse inhibition (PPI) relative to civilians. PPI occurs when a relatively weak sensory event (prepulse) is presented just before a strong startle inducing stimulus and reduces the magnitude of the startle response (Grillon et al. Psychiatry Res. 1996. 64:169-178). PPI is thought to occur through a sensorimotor gating system that functions as an attentional filter to protect limited capacity systems from being overloaded with incoming sensory information. Increased PPI is thought to be associated with more effective information processing, whereas reduced PPI is thought to reflect reduced efficiency in inhibiting information. Thus, impaired PPI may reflect a reduced ability to inhibit sensory information and potentially a failure of sensorimotor gating. Studies show reduced PPI in individuals with PTSD. Lower PPI is also associated with higher overall PTSD severity, reexperiencing and hyperarousal symptoms (Echiverri-Cohen et al. J Anxiety Disord. 2016. 37:94-103).

The inventors of the present invention have also discovered an additional alternative measure of exaggerated startle response, which is the intrinsic auricular muscle response (IAMR). Intrinsic auricular muscles comprise helicis major, helicis minor, tragicus and antitragicus muscles. It is known that the tragicus and antitragicus muscles cooperate to constrict the external auditory meatus as the orbicularis oculi muscle constricts the palpebral fissure (Matsuo and Hiroshe. Eur J Plast Surg. 1987. 10:82-83). This is thought to be due to the eyeblink pathway wherein eyelid movement is generated by the facial motor nucleus (Freeman and Steinmetz. Learn Mem. 2011. 18:666-677). Facial nerve additionally innervates the muscles of the auricle. As a result, signals for movement of the orbicularis oculi transmitted by the facial nerve while blinking also reach the tragicus and antitragicus muscles, causing the constriction of the external auditory meatus. Therefore it is possible to determine the startle response by measuring the intrinsic auricular muscle response (IAMR) of the subject by electrodes on the skin surface over the muscles. It is also possible to use helicis major, helicis minor muscles for this purpose.

A system for determining PTSD using eyeblink response, post-auricular muscle response (PAMR) and instrinsic auricular muscle response (IAMR), is disclosed in US 2019/0008435 (A1).

The present disclosure aims to provide a system whereby the acoustic startle reflex, namely pupillary response, IAMR and PAMR, of a subject and change in PPI of a subject are measured in order to present an effective and inexpensive method for diagnosing PTSD. The system comprises earphones for delivering auditory stimuli to the subject and a display device, such as a computer screen or a mobile phone for supplying pleasant and/or unpleasant prepulse stimuli to the subject. The earphones additionally contain electrodes for measurement of PAMR and IAMR respectively. The system also contains pupillary response measuring means for determining the pupillary response of the subject and measuring the amplitude thereof.

The present invention provides a diagnostic system as defined in Claim 1.

### Objects of the Present Invention

The object of the invention is defined in claim 1. It aims to provide a system whereby the acoustic startle reflex of a subject is measured in order to present an effective and inexpensive method for diagnosing PTSD.

Another object of the invention is to provide a system whereby the lateralization of the pupillary response to stimuli is measured and used as a marker in order to present an effective and inexpensive method for diagnosing PTSD.

Another object of the invention is to provide a system whereby startle pupillary response, PAMR and IAMR and change in PPI of a subject are simultaneously measured and analyzed in order to present a corroborative, effective and inexpensive method for diagnosing PTSD.

An alternative object of the disclosure is to provide a system whereby the lateralization of the pupillary response to stimuli is measured and used as a marker in order to present an effective and inexpensive method for determining the possible location of the damage to brain stem pathways.

### Brief Description of the Technical Drawings

Accompanying drawings are given solely for the purpose of exemplifying a diagnostic system, whose advantages over prior art were outlined above and will be explained in brief hereinafter.

The drawings are not meant to delimit the scope of protection as identified in the Claims, nor should they be referred to alone in an effort to interpret the scope identified in said Claims without recourse to the technical disclosure in the description.
Figures 1A and 1B demonstrate anatomical structures of the human head relevant to the present invention.
Figure 2 demonstrates a block diagram of a diagnostic system according to the present invention.
Figure 3 demonstrates a schematic view of one embodiment of a diagnostic system according to the present invention.

### Detailed Description

The following numerals are referred to in the detailed description of the present invention:
- 10: Subject
- 11: Pinna
- 12: Post-auricular muscle
- 13: Tragicus muscle
- 14: Antitragicus muscle
- 15: Eye of subject
- 16: Pupil
- 17: Iris sphincter muscle
- 18: Iris dilator muscle
- 100: Diagnostic system
- 110: Stimuli delivery module
- 111: Sensor module
- 112: Diagnosis module
- 120: Startle stimuli
- 121: Prepulse stimuli
- 130: Pupillary response
- 131: Post-auricular muscle response
- 132: Intrinsic auricular muscle response
- 200: Ear-wearable body
- 201: Earphones
- 202: Measurement portion
- 203: Smartphone
- 204: Screen
- 205: Camera

A recent study has shown that, veterans higher in PTSD reports also showed significantly larger pupils to threatening pictures. Studies have also found increased pupil size to arousing pictures and emotional auditory stimuli. Data from the study suggest increased autonomic arousal in those with higher PTSD symptoms when exposed to threatening stimuli, which is consistent with the clinical presentation of PTSD (Kimble, et al. J Anxiety Disord. 2010. 24:293-299). Another study has shown that individuals with PTSD showed significantly more pupil dilation to threat-relevant stimuli compared to the neutral figural elements, and to trauma-exposed controls. Arousal-related pupil dilation significantly predicted PTSD after time elapsed since trauma, cumulative violence exposure, and trait anxiety were statistically controlled. (Cascardi, et al. J Trauma Stress. 2015. 28:370-374). Therefore, pupillometry can be a valuable addition to the study of dysregulated autonomic arousal after trauma.

As mentioned above, pupil size is determined by a balancing act between two smooth muscles: the sphincter and dilator muscles of the iris. The dilator muscle is innervated by sympathetic neurons in the superior cervical ganglion (SCG), which is, in turn, innervated by the intermediolateral cell column (IML) of the spinal cord. The sphincter muscle is innervated by parasympathetic neurons in the ciliary ganglion (CG), which is, in turn, innervated by the Edinger-Westphal nucleus (EWN). Thus, either excitation of the sympathetic SCG neurons or inhibition of the parasympathetic EWN neurons results in pupil dilation. Studies have shown that unilateral LC stimulation evoked pupil dilation. It was shown that unilateral LC activation evoked bilateral dilation and an across-eye difference in the size of the evoked bilateral dilation, indicating a lateralization in the pathways between the LC and both pupils. It was found that the LC influences the ipsilateral pupil through both parasympathetic and sympathetic pathways but influences the contralateral pupil only through the parasympathetic pathway. Therefore, differences between bilateral pupil dilations were mainly attributed to sympathetic contribution, suggesting that a measurement of across-eye differences in bilateral pupil dilation may present itself as a biomarker for abnormal autonomic activities (Liu, et al. Cell Reports. 2017. 20:3099-3112.). A recent study has also shown that there is a link between LC hyperactivity and PTSD (Naegeli, Christoph, et al. Biol Psychiatry. 2018. 83:254-262).

Therefore, the lateralization of the pupillary response can be used as a marker for the diagnosis of PTSD. Alternatively, the lateralization of the pupillary response can be used to determine the possible location of the damage to brain stem pathways.

When an individual is exposed to an unpleasant stimulus, the relevant subcortical, aversive system circuitry is activated which leads to the augmentation of responses such as the eyeblink response, pupillary response, skin conductance responses and other startle response described above.

The pupillary response can start emerging as early as 200 ms after stimulus onset and is modulated by arousal: the pupil has been shown to dilate more strongly in response to emotionally arousing picture stimuli as well as to arousing sounds in comparison to neutral stimuli. The conditioned pupil response is often defined as the increase in diameter from stimulus onset to offset, spanning several seconds. Besides responding to arousal, the pupil has been shown to be sensitive to a variety of higher-order cognitive processes such as error monitoring, uncertainty, or cognitive load. It is yet unknown how pupil responses relate to other physiological readouts of conditioned fear like startle responses (Leuchs, Schneider and Spoormaker. Psychophysiology. 2019. 56:e13283). Therefore an additional advantage of the present invention is correlating known modes of startle responses such as PAMR and IAMR with pupillary response.

The magnitude of startle responses are modified if the startle-eliciting noise is preceded by the occurrence of a transient stimulus, an effect known as prepulse inhibition (PPI). PPI is thought to reflect a low-level sensory gating mechanism by which excess or trivial stimuli are screened or "gated out" of awareness, so that an individual can focus attention on the most salient aspects of the stimulus-laden environment. PPI is a ubiquitous phenomenon and can occur when the prepulse and startling stimuli are in the same or different sensory modalities (Benning et al. Psychophysiology 2004. 41:426-432; Braff et al. Psychopharmacology. 2001. 156:234-258).

Patients with PTSD exhibit normal acoustic startle amplitude, but show a reduction in PPI relative to the non-affected subjects. PPI deficits are associated with perceptual abnormalities and deficiencies in gating irrelevant thoughts. Patients with PTSD are also characterized by perceptual deficits and by an inability to gate intrusive thoughts. Some of the symptoms of PTSD such as the intrusion of unwanted thoughts (e.g. flashback) could be hypothesized to result from a gating deficit. Alternatively, reduced PPI in patients with PTSD can reflect an attentional deficit. PPI is increased with increased attention to the prepulse and reduced PPI in patients with PTSD suggests less processing of the prepulse, perhaps because of distraction by internal stimuli (e.g. thoughts) (Grillon et al. Psychiatry Res. 1996. 64:169-178).

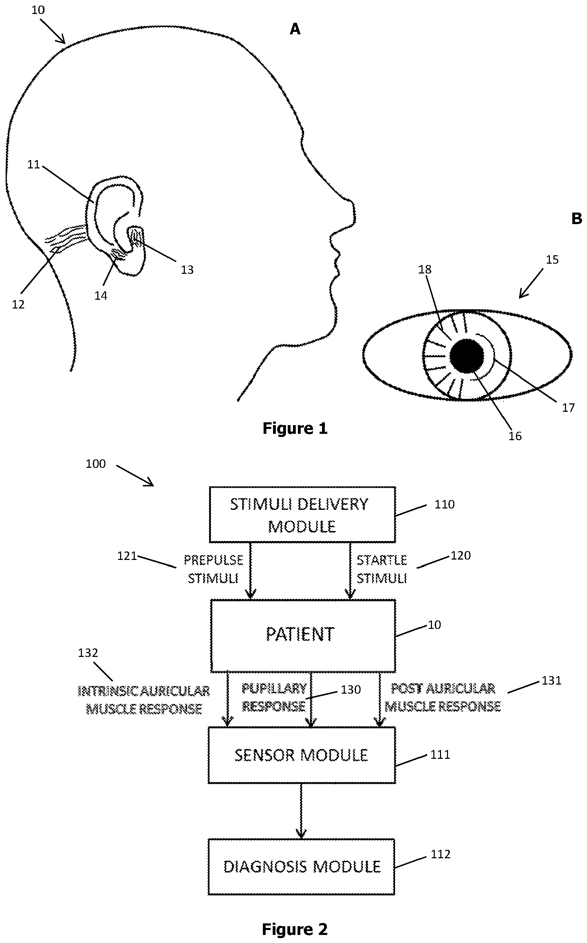

The present invention discloses a diagnostic system (100) comprising a stimuli delivery module (110), connected to earphones and at least one display, whereby startle stimuli (120) and prepulse stimuli (121) are delivered to subject (10); at least one sensor module (111), connected to two electrodes and one pupillary response measuring means, whereby the startle response, i.e. the pupillary response (130), PAMR (131) and IAMR (132), and PPI of subject (10) are detected and recorded; and a diagnosis module (112) whereby the features of the startle response and PPI of subject (10) are extracted and classified by a software application (not shown) as will be delineated hereinafter (Figure 2).

As mentioned above, PAMR (131) is a large and simply measured auditory response, (up to 8 kHz or higher). The response is optimally recorded as the potential difference between the skin overlying the post-auricular muscle (12) and the rear of the pinna (11) of the subject (10), with a bandwidth from 10 to 300 Hz. This electrode positioning also reduces background electrical interference and muscle activity and eliminates blink artefacts (O'Beirne and Patuzzi. Hearing Res. 1999. 138:115-132).

As also mentioned above, IAMR (132) can be used as an alternative measure of exaggerated startle response. The response can be recorded by placing electrodes on the tragicus (13) and antitragicus muscles (14) of the subject (10). In an alternative embodiment, IAMR (132) can be recorded by placing electrodes on the helicis major, helicis minor of the subject (10).

The diagnostic system (100) measures the auditory startle response (130, 131, 132) of a subject (10). Auditory startle response (130, 131, 132) is measured by stimuli delivery module (110) by delivering startle stimuli (120) to subject (10). Startle stimuli (120) are delivered by playing a sudden, loud tone or noise, such as a sound burst of 95 decibels, for subject (10) preferably using earphones. The diagnostic system (100) additionally measures the PPI of a subject (10) by delivering prepulse stimuli (121) to a subject (10) shortly before delivering startle stimuli (120). Prepulse stimuli (121) are delivered by showing pleasant, unpleasant and neutral visuals to subject (10) via a display. Display includes monitors, TV screens, flat panel displays, video walls, or any other type of display device to display the prepulse stimuli (121) to subject (10). In one embodiment, visuals are selected from the International Affective Picture System (IAPS) to represent the different valence (pleasant, unpleasant and neutral) categories. Preferably, startle stimuli (120) is delivered to the subject (10) 3-5 s after the delivery of prepulse stimuli (121).

The present invention makes use of the lateralization of pupillary response (130) as a biomarker for the diagnosis of PTSD. In a preferred embodiment of the invention, startle stimuli (120) is delivered unilaterally using earphones. Namely, startle stimuli (120) is delivered via the right ear or left ear and the bilateral pupillary response (130) is monitored. Alternatively, the present disclosure makes use of the lateralization of pupillary response (130) as a biomarker for damages to the brain stem of a subject. By measuring the difference in the ipsilateral and contralateral response to startle stimuli (120) in each eye, the possible location of the damage to brain stem pathways may be assessed.

Diagnostic system (100) also includes a sensor module (111) comprising plurality of sensors (not shown). The sensors are configured to monitor and record a plurality of different physiological responses to subject's (10) exposure to stimuli output (120, 121), such as pupillary response (130), PAMR (131) and IAMR (132). The physiological response data are collected during the subject's (10) exposure to the stimuli.

Sensor module (111) detects the subject's (10) response to startle stimuli (120) by monitoring the subject's (10) pupillary response (130). This can be achieved by any method present in the art, such as a camera or a desktop mounted eye-tracking system, such as Eyelink-1000 (SR research, Kanata, Ontario, Canada).

Sensor module (111) also comprises at least one electrode designed to be placed on the back of the ear corresponding to post-auricular muscle (12) to measure the PAMR (131) of subject (10) simultaneously while measuring the pupillary response (130), thereby increasing accuracy. Preferably, sensor module (111) comprises two electrodes to be placed one on the back of the ear of subject (10) directly over post-auricular muscle (12) and one directly adjacent on pinna (11) of subject (10) and a grounding electrode to be placed elsewhere on subject's (10) head. The PAMR (131) may be determined on one side, either ipsilateral or contralateral, of the subject (10), or on both sides of the subject (10).

Additionally, sensor module (111) comprises a sensor such as an EMG sensor detects the electric potential generated by the tragicus muscle (13) and antitragicus muscle (14) in order to measure the IAMR (132) of the subject. The sensor may comprise surface electrodes (surface EMG) or needle-shaped electrodes (intramuscular EMG). In one embodiment of the invention, needle-shaped electrodes are pricked on the auricular skin and reaches said tragicus muscle (13) and antitragicus muscle (14).

The pupillary response (130), PAMR (131), IAMR (132) and PPI data are collected by sensor module (111) and transmitted to diagnosis module (112). Diagnosis module (112) can be in wired or wireless communication with sensor module (111). Diagnosis module (112) comprises a software application for analyzing data which can include the amplitude and speed of response (130, 131, 132) and change in PPI to determine whether the individual is suffering from PTSD. Alternatively, diagnosis module (112) comprises a software application for analyzing data including the amplitude and speed of pupillary response (130) of a subject to determine the possible location of the damage to brain stem pathways of said subject.

Diagnosis module (112) may also determine a threshold value depending on at least one of the age, ethnic background, sex and baseline response of subject (10). In some embodiments, diagnosis module (112) combines and applies weights to the processed extracted features and compares the combined value to the threshold. The weighted features can be combined by any arithmetic process. In such embodiments, diagnosis module (110) identifies subject (10) as having PTSD if the combined value is above the threshold, and not having PTSD if the combined value is below the threshold.

In some embodiments, diagnostic system (100) may be coupled to a network interface configured for wired or wireless data communications, and the results may be transmitted to a remote computing system over a computer network and displayed to a clinician, care provider and/or subject (10) by outputting the results via a display device concurrently or after the fact.

In some embodiments, diagnostic system (100) can comprise a mobile electronic device, such as a smartphone, tablet, PC computer or any other like device known in the art.

Diagnostic system (100) allows startle response (130, 131, 132) and PPI to be monitored over time (long-term monitoring) and also emotional state of the subject (10) at that moment to be determined.

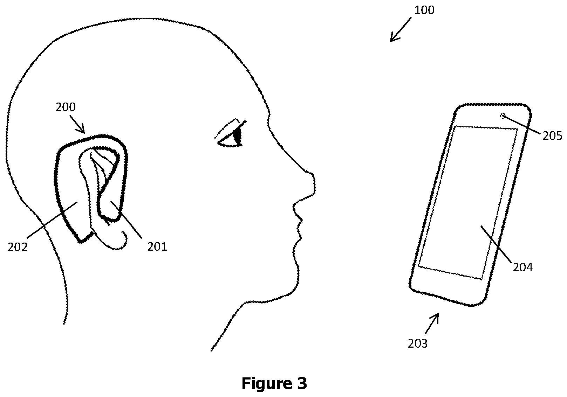

Figure 3 illustrates an embodiment of diagnostic system (100). Diagnostic system (100) comprises an ear-wearable body (200) equipped with earphones (201) and a measurement portion (202) and a smartphone (203) equipped with a screen (204) and a built-in camera (205) a mobile application (not shown) for the analysis of data.

Earphones (201) are used to deliver startle stimuli (120) to subject (10) and screen (204) is used to deliver prepulse stimuli (121) to subject (10). In alternative embodiments, display may be the screen (204) of a smartphone or other mobile device, tablet, computer.

Measurement portion (202) comprises electrodes designed to be placed on the skin of the back of the ear of subject (10) corresponding to the post-auricular muscle (12) whereby the detection of the PAMR (131) of the subject (10) is realized as described above. Measurement portion (202) additionally comprises electrodes designed to be placed on the skin of the entrance of the ear canal of subject (10) corresponding to the tragicus muscle (13) and antitragicus muscle (14) the detection of the IAMR (132) of the subject (10) is realized.

Camera (205) is used to record the pupil size of subject (10) to determine the pupillary response (130) to startle stimuli (120).

## Claims

1. A diagnostic system (100) for measuring and analyzing startle response of a subject (10), wherein
said diagnostic system (100) comprises a stimuli delivery module (110), whereby auditory startle stimuli (120) and prepulse stimuli (121) are delivered to subject (10);
said diagnostic system (100) comprises at least one sensor module (111), whereby the auditory startle response (130, 131, 132) of subject (10) are detected and recorded;
said sensor module (111) comprises at least two electrodes and one pupillary response measuring means;
said diagnostic system (100) comprises a diagnosis module (112) whereby the features of the auditory startle response (130, 131, 132) and prepulse inhibition of subject (10) are extracted and analyzed;
said sensor module (111) is configured to detect the startle response of subject (10) comprising pupillary response (130), post-auricular muscle response (131) and intrinsic auricular muscle response (132);
said stimuli delivery module (110) comprises a set of earphones whereby auditory startle stimuli (120) is delivered to subject (10);
said stimuli delivery module (110) comprising the set of earphones is configured to deliver auditory startle stimuli (120) to subject (10) unilaterally; and
said sensor module (111) comprising the pupillary response measuring means is configured to facilitate the measurement of ipsilateral and contralateral pupillary response (130) to unilateral auditory startle stimuli (120).

2. A diagnostic system (100) as set forth in Claim 1, wherein said startle stimuli (120) is a sudden, loud tone or noise.

3. A diagnostic system (100) as set forth in Claim 2, wherein said startle stimuli (120) is a sound burst of 95 decibels.

4. A diagnostic system (100) as set forth in Claim 1, wherein stimuli delivery module (110) comprises a display whereby prepulse stimuli (121) is delivered to subject (10).

5. A diagnostic system (100) as set forth in Claim 1, wherein said prepulse stimuli (120) are pleasant, unpleasant and neutral visuals.

6. A diagnostic system (100) as set forth in Claim 5, wherein said prepulse stimuli (120) are pleasant, unpleasant and neutral visuals selected from the International Affective Picture System (IAPS).

7. A diagnostic system (100) as set forth in Claim 1, wherein said sensor module (111) comprises at least one electrode configured to be placed over tragicus (13) and antitragicus muscles (14) of subject (10) whereby measurement of intrinsic auricular muscle response (132) of subject (10) is facilitated.

8. A diagnostic system (100) as set forth in Claim 1, wherein said sensor module (111) comprises at least one electrode configured to be placed over helicis major and helicis minor of subject (10) whereby measurement of intrinsic auricular muscle response (132) of subject (10) is facilitated.

9. A diagnostic system (100) as set forth in Claim 1, wherein said sensor module (111) comprises at least one electrode configured to be placed on the scalp behind the ear of subject (10) whereby measurement of post-auricular muscle response (131) of subject (10) is facilitated.

10. A diagnostic system (100) as set forth in Claim 9, wherein said sensor module (111) comprises one electrode configured to be placed on the back of the ear of subject (10) directly over post-auricular muscle (12) and one electrode configured to be placed directly adjacent on pinna (11) of subject (10).

11. A diagnostic system (100) as set forth in Claim 9, wherein said sensor module (111) comprises a grounding electrode.

12. A diagnostic system (100) as set forth in Claim 1, wherein said pupillary response measuring means is a camera whereby measurement of pupillary response (130) of subject (10) is facilitated.

13. A diagnostic system (100) as set forth in Claim 1, wherein said sensor module (11) and said diagnosis module (112) are in wired or wireless communication.

14. A diagnostic system (100) as set forth in Claim 13, wherein said diagnosis module (112) comprises a software application,
said diagnosis module (112) comprises a software application whereby feature extraction and analysis of startle response (130, 131, 132) data is performed,
said startle response (130, 131, 132) data comprises the amplitude and speed of response (130, 131, 132) and change in prepulse inhibition,
said startle response (130, 131, 132) data comprises the lateralization of pupillary response (130) to unilaterally applied startle stimuli (120),
said diagnostic system (100) is in wired or wireless communication with a network interface whereby said feature extraction and analysis results are communicated to a clinician, care provider and/or subject (10), and
said diagnostic system (100) is in wired or wireless communication with a network interface whereby said feature extraction and analysis results are outputted via a display device concurrently or after the fact.

15. A diagnostic system (100) as set forth in any preceding Claim, wherein said diagnostic system (100) comprises a mobile electronic device, such as a smartphone, tablet or PC computer.

## Patentansprüche

1. Diagnosesystem (100) für die Messung und Analyse der Schreckreaktion eines Subjekts (10)**, wobei**;
das Diagnosesystem (100) ein Reizzuführmodul (110) aufweist, wodurch dem Subjekt (10) auditorische Schreckreizen (120) und Präpulsreizen (121) zugeführt werden;
das Diagnosesystem (100) mindestens ein Sensormodul (111) aufweist, wodurch die auditorische Schreckreaktion (130, 131, 132) des Subjekts (10) erkannt und aufgenommen wird;
das Sensormodul (111) mindestens zwei Elektroden und ein Mittel für die Messung der Pupillenreaktion aufweist;
das Diagnosesystem (100) ein Diagnosemodul (112) aufweist, wodurch die Merkmale der auditorischen Schreckreaktion (130, 131, 132) und der Präpulsinhibition des Subjekts (10) gewonnen und analysiert werden;
das Sensormodul (111) ausgelegt ist, um die Schreckreaktion des Subjekts (10) einschließlich die Pupillenreaktion (130), die Reaktion des postaurikulären Muskels (131) und die Reaktion des intrinsischen aurikulären Muskels (132) zu erkennen;
das Reizzuführmodul (110) einen Satz von Ohrhörern aufweist, wodurch dem Subjekt (10) auditorische Schreckreizen (120) zugeführt werden;
das Reizzuführmodul (110), das den Satz von Ohrhörern aufweist ausgelegt ist, die auditorischen Schreckreizen (120) unilateral dem Subjekt (10) zuzuführen; und
das Sensormodul (111) das das Mittel für die Messung der Pupillenreaktion aufweist ausgelegt ist, die Messung der ipsilateralen und kontralateralen Pupillenreaktion (130) auf unilaterale auditorische Schreckreizen (120) zu erleichtern.

2. Diagnosesystem (100) gemäß Anspruch 1, **wobei** der Schreckreiz (120) ein plötzlicher, lauter Ton oder Krach ist.

3. Diagnosesystem (100) gemäß Anspruch 2, **wobei** der Schreckreiz (120) eine Klang Böe von 95 Dezibels ist.

4. Diagnosesystem (100) gemäß Anspruch 1, **wobei das** Reizzuführmodul (110) ein Anzeigemodul aufweist, wodurch dem Subjekt (10) Präpulsreizen (121) zugeführt werden.

5. Diagnosesystem (100) gemäß Anspruch 1, **wobei** die Präpulsreizen (121) angenehme, unangenehme und neutrale visuelle Reizen sind.

6. Diagnosesystem (100) gemäß Anspruch 5, **wobei** die Präpulsreizen (121) aus dem International Affective Picture System (IAPS) ausgewählte angenehme, unangenehme und neutrale visuelle Reizen sind.

7. Diagnosesystem (100) gemäß Anspruch 1, **wobei** das Sensormodul (111) mindestens eine Elektrode aufweist, die ausgelegt ist, um über die Tragusmuskeln (13) und Antitragusmuskeln (14) des Subjekts (10) platziert zu werden, wodurch die Messung der Reaktion des inneren aurikulären Muskels (132) des Subjekts (10) erleichtert wird.

8. Diagnosesystem (100) gemäß Anspruch 1, **wobei** das Sensormodul (111) mindestens eine Elektrode aufweist, die ausgelegt ist, um über die Helicis Major und Helicis Minor des Subjekts (10) platziert zu werden, wodurch die Messung der Reaktion des inneren aurikulären Muskels (132) des Subjekts (10) erleichtert wird.

9. Diagnosesystem (100) gemäß Anspruch 1, **wobei** das Sensormodul (111) mindestens eine Elektrode aufweist, die ausgelegt ist, um auf der Kopfhaut hinter dem Ohr des Subjekts (10) platziert zu werden, wodurch die Messung von Reaktionen des postaurikulären Muskels (131) des Subjekts (10) erleichtert wird.

10. Diagnosesystem (100) gemäß Anspruch 9, **wobei** das Sensormodul (111) eine Elektrode aufweist, die ausgelegt ist, um hinter dem Ohr des Subjekts (10) unmittelbar über den postaurikulären Muskel (12) platziert zu werden und eine Elektrode aufweist die ausgelegt ist, um unmittelbar neben der Ohrmuschel (11) des Subjekts (10) platziert zu werden.

11. Diagnosesystem (100) gemäß Anspruch 9, **wobei** das Sensormodul (111) eine Erdungselektrode aufweist.

12. Diagnosesystem (100) gemäß Anspruch 1, **wobei** das Mittel für die Messung der Pupillenreaktion eine Kamera ist, wodurch die Messung der Pupillenreaktion (130) des Subjekts (10) erleichtert wird.

13. Diagnosesystem (100) gemäß Anspruch 1, **wobei** das Sensormodul (111) und das Diagnosemodul (112) sich in drahtgebundener oder drahtloser Kommunikation befinden.

14. Diagnosesystem (100) gemäß Anspruch 13, **wobei** das Diagnosemodul (112) eine Softwareanwendung aufweist,
das Diagnosemodul (112) eine Softwareanwendung aufweist, wodurch die Gewinnung und Analyse von Merkmalsdaten der Schreckreaktion (130, 131, 132) durchgeführt wird,
die Daten der Schreckreaktion (130, 131, 132) die Amplitude und die Geschwindigkeit der Reaktion (130, 131, 132) sowie die Veränderung der Präpulsinhibition umfassen, die Daten der Schreckreaktion (130, 131, 132) die Lateralisierung der Pupillenreaktion (130) auf unilateral angelegte Schreckreizen (120) umfassen,
das Diagnosesystem (100) sich in drahtgebundener oder drahtloser Kommunikation mit einer Netzwerkschnittstelle befindet, wodurch die Ergebnisse über die Gewinnung und die Analyse der Merkmalen einem Arzt, einem Pfleger und/oder dem Subjekt (10) vermittelt werden, und
das Diagnosesystem (100) sich in drahtgebundener oder drahtloser Kommunikation mit einer Netzwerkschnittstelle befindet, wodurch die Ergebnisse über die Gewinnung und die Analyse von Merkmalen gleichzeitig oder nachträglich über ein Anzeigegerät ausgegeben werden.

15. Diagnosesystem (100) gemäß irgendeinem der vorhergehenden Ansprüche, wobei das Diagnosesystem (100) ein mobiles elektronisches Gerät wie ein Smartphone, ein Tablet oder ein PC-Computer aufweist.

## Revendications

1. Système diagnostique (100) pour mesurer et analyser la réaction de sursaut d'un sujet (10);
ledit système diagnostique (100) comportant un module de délivrance de stimuli (110), par lequel des stimuli auditifs de sursaut (120) et des stimuli de pré-impulsion (121) sont délivrés au sujet (10) ;
ledit système diagnostique (100) comportant au moins un module capteur (111), par lequel la réaction de sursaut (130, 131, 132) du sujet (10) est détectée et enregistrée; ledit module capteur (111) comportant au moins deux électrodes et un moyen de mesure de la réaction pupillaire ;
ledit système diagnostique (100) comportant un module de diagnostic (112) par lequel les caractéristiques de la réaction de sursaut auditive (130, 131, 132) et de l'inhibition de pré-impulsion du sujet (10) sont récupérées et analysées ;
ledit module capteur (111) étant conçu pour détecter la réaction de sursaut du sujet (10 ) y compris la réaction pupillaire (130), la réaction du muscle auriculaire postérieur (131) et la réaction du muscle intrinsèque auriculaire (132) ;
ledit module de délivrance de stimuli (110) comportant un ensemble d'écouteurs par lesquels des stimuli auditifs de sursaut (120) sont délivrés au sujet (10) ;
ledit module de délivrance de stimuli (110) comportant l'ensemble d'écouteurs étant conçu pour délivrer des stimuli auditifs de sursaut (120) au sujet (10) de manière unilatérale ; et
ledit module capteur (111) comportant le moyen de mesure de la réaction pupillaire étant conçu pour faciliter la mesure de la réaction pupillaire ipsilatérale et controlatérale (130) à des stimuli de sursaut auditifs unilatéraux (120).

2. Système diagnostique (100) selon la revendication 1, **dans lequel** le stimulus de sursaut (120) est un ton ou un bruit soudain et fort.

3. Système diagnostique (100) selon la revendication 2, **dans lequel** ledit stimulus de sursaut (120) est un éclat sonore de 95 décibels.

4. Système diagnostique (100) selon la revendication 1, **dans lequel le** module de délivrance de stimuli (110) comprend un écran par lequel des stimuli de pré-impulsion (121) sont délivrés au sujet (10).

5. Système diagnostique (100) selon la revendication 1, **dans lequel** les stimuli de pré-impulsion (121) sont des visuels plaisants, déplaisants et neutres.

6. Système diagnostique (100) selon la revendication 5, **dans lequel** les stimuli de pré-impulsion (121) sont des visuels plaisants, déplaisants et neutres sélectionnées dans le système international d'images affectives (IAPS).

7. Système diagnostique (100) selon la revendication 1, **dans lequel** ledit module capteur (111) comporte au moins une électrode conçue pour être placée sur les muscles tragus (13) et antitragus (14) du sujet (10), par laquelle le mesurage de la réaction du muscle intrinsèque auriculaire (132) du sujet (10) est facilité.

8. Système diagnostique (100) selon la revendication 1, **dans lequel** ledit module capteur (111) comporte au moins une électrode conçue pour être placée sur l'hélice majeur et l'hélice mineur du sujet (10), par laquelle le mesurage de la réaction du muscle intrinsèque auriculaire (132) du sujet (10) est facilité.

9. Système diagnostique (100) selon la revendication 1, **dans lequel** ledit module capteur (111) comporte au moins une électrode conçue pour être placée sur le scalp derrière l'oreille du sujet (10) par laquelle le mesurage de la réaction du muscle auriculaire postérieur (131) du sujet (10) est facilité.

10. Système diagnostique (100) selon la revendication 9, **dans lequel** ledit module capteur (111) comporte une électrode conçue pour être placée en arrière de l'oreille du sujet (10) directement sur le muscle auriculaire postérieur (12) et une électrode conçue pour être placée directement adjacente au pavillon de l'oreille (11) du sujet (10).

11. Système diagnostique (100) selon la revendication 9, **dans lequel** ledit module capteur (111) comporte une électrode de terre.

12. Système diagnostique (100) selon la revendication 1, **dans lequel** le moyen de mesure de la réaction pupillaire est une caméra par lequel le mesurage de la réaction pupillaire (130) du sujet (10) est facilité.

13. Système diagnostique (100) selon la revendication 1, **dans lequel** ledit module capteur (111) et ledit module de diagnostic (112) sont en communication filaire ou sans fil.

14. Système diagnostique (100) selon la revendication 13, **dans lequel** ledit module de diagnostic (112) comporte une application logicielle,
ledit module de diagnostic (112) comportant une application logicielle permettant de récupérer les caractéristiques et d'analyser les données relatives à la réaction de sursaut (130, 131, 132),
les données relatives à la réaction de sursaut (130, 131, 132) incluant l'amplitude et la rapidité de la réaction (130, 131, 132) et le changement dans l'inhibition de la pré-impulsion,
lesdites données relatives à la réaction de sursaut (130, 131, 132) incluant la latéralisation de la réaction pupillaire (130) à des stimuli de sursaut appliqués unilatéralement (120),
ledit système diagnostique (100) étant en communication filaire ou sans fil avec une interface de réseau par laquelle les résultats de la récupération et de l'analyse des caractéristiques sont communiqués à un clinicien, à un soignant et/ou à un sujet (10), et
ledit système diagnostique (100) étant en communication filaire ou sans fil avec une interface de réseau par laquelle les résultats de la récupération et de l'analyse des caractéristiques sont émis simultanément ou rétrospectivement par un appareil d'affichage.

15. Système diagnostique (100) selon l'une quelconque des revendications précédentes, dans lequel ledit système diagnostique (100) comporte un appareil électronique mobile, tel qu'un smartphone, une tablette ou un ordinateur PC.
